(19) Europäisches Patentamt
European Patent Office
Office européen des brevets

(11) Publication number: 0 163 506
B1

(12) # EUROPEAN PATENT SPECIFICATION

(45) Date of publication of patent specification: 02.11.89

(51) Int. Cl.$^4$: C 07 D 213/64

(21) Application number: 85303709.1

(22) Date of filing: 28.05.85

(54) **Process for the preparation of a pyridil-propanoic acid.**

(30) Priority: 31.05.84 GB 8413925

(43) Date of publication of application:
04.12.85 Bulletin 85/49

(45) Publication of the grant of the patent:
02.11.89 Bulletin 89/44

(84) Designated Contracting States:
AT BE CH DE FR GB IT LI LU NL SE

(58) References cited:

None

(73) Proprietor: SMITH KLINE & FRENCH LABORATORIES LIMITED
Mundells
Welwyn Garden City Hertfordshire, AL7 1EY (GB)

(72) Inventor: Adger, Brian Michael
8 Tonbridge Road Hildenborough
Nr Tonbridge Kent TN9 2NG (GB)
Inventor: Lewis, Norman John
20A Norton Road Southborough
Tunbridge Wells Kent TN4 0HE (GB)

(74) Representative: Denerley, Paul Millington, Dr. et al
Smith Kline & French Laboratories Ltd Patent Department Mundells
Welwyn Garden City Hertfordshire AL7 1EY (GB)

EP 0 163 506 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for preparing 3-(pyrid-4-yl)propanoic acids and salts thereof.

EP—A—3677, 4793, 13071, 15138, 17679 and 17680 disclose *inter alia* compounds of the general formula:

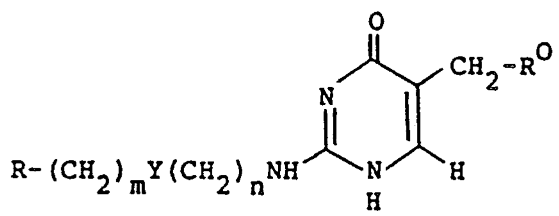

(I)

wherein R represents various optionally substituted heteroaryl groups or optionally substituted phenyl; $-(CH_2)_mY(CH_2)_n-$ represents a range of linking chains known in the histamine antagonist art; and $R^0$ is optionally substituted pyridyl. These compounds are useful histamine atagonists.

The 3-(pyrid-4-yl)propanoic acids which can be prepared by the process of the present invention can be used as intermediates in preparing certain compounds of the formula (I).

Accordingly the present invention provides a process for preparing a compound of the formula (II):

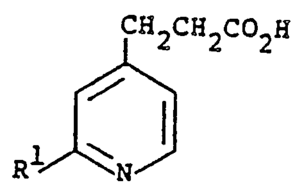

(II)

or a salt thereof, wherein $R^1$ is $C_{1-6}$alkoxy, benzyloxy or p-nitrobenzyloxy, which comprises reacting an anion of the formula (III):

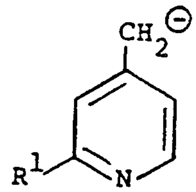

(III)

wherein $R^1$ is as defined in relation to formula (II), with a compound of the formula (IV):

$$X-CH_2CO_2^-M^+ \qquad \text{(IV)}$$

wherein X is a leaving group and $M^+$ is a counter-ion, and if desired interconverting an acid of the formula (II) with a salt thereof.

We have surprisingly found that an anion of the formula (III) wherein $R^1$ is methoxy and a compound: $X-CH_2CO_2C_2H_5$ wherein X is chloro or bromo did not react. Furthermore when reacting a compound of the formula (IV) wherein X is chloro with an anion corresponding to formula (III), wherein $R^1$ is replaced by chloro, no desired product resulted. Therefore it is unexpected and advantageous that the reaction of the present invention is successful in good yields and purity.

Examples of $R^1$ are methoxy, ethoxy, n-propoxy and n-butoxy; benzyloxy and p-nitrobenzyloxy.

Preferably $R^1$ is methoxy or benzyloxy.

Therefore suitable anions of the formula (III) for use in this invention are those formed from 2-methoxy-4-methylpyridine, 2-ethoxy-4-methylpyridine and 2-benzyloxy-4-methylpyridine.

In the compounds of the formula (IV) favourably X is halo, for example chloro, bromo or iodo; preferably X is chloro or bromo.

The counter-ion $M^+$ may be any convenient counter-ion, for example an alkali metal ion such as lithium, sodium or potassium.

The anion of the compound of the formula (III) has a counter-ion which can be, for example, lithium, sodium or potassium, preferably sodium or potassium.

The reaction between an anion of the formula (III) and a compound of the formula (IV) is performed in the presence of a solvent which is substantially inert to the reagents and products. The anion of the compound of the formula (III) can be prepared in situ by reacting a 4-methylpyridine precursor compound

with an alkali metal amide, for example sodamide. Preferably in such a case the solvent is liquid ammonia, which optionally may be in admixture with a co-solvent, such as tetrahydrofuran or dioxan. The reaction can also be performed in the presence of a phase transfer catalyst. Alternatively the anion can be prepared in situ with an alkyl alkali, for example butyl lithium. In a further alternative the anion can be prepared with lithium di-isopropylamide. Preferably in such cases the solvent is an ether for example diethyl ether or tetrahydrofuran.

Preferably the anion of the formula (III) is prepared *in situ* and to this is added the compound of the formula (IV).

The reaction preferably is performed at a moderate to low temperature. For example when the anion of the formula (III) is generated in situ using an alkali metal amide in liquid ammonia, the reaction is carried out at or below the boiling point of liquid ammonia. When the anion of the formula (III) is generated *in situ* from an alkyl alkali the reaction is carried out between room temperature and −78°C, preferably under an inert atmosphere.

The compound of the formula (II) may be isolated either in acid or salt form, or converted from one form to the other, in conventional manner.

The compounds of the formula (II) are useful as they can be esterified and formylated in conventional manner to give valuable intermediates (V) for the preparation of compounds within the formula (I):

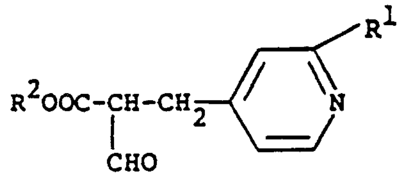

(V)

wherein $R^1$ is as hereinbefore defined and $R^2$ is $C_{1-6}$alkyl. Formylation can be performed for example as described in EP—A—3677. The use of the compounds of the formula (V) is described in the aforementioned European Patent Applications.

The following Examples illustrate this invention.

Example 1

3-(2-Benzyloxypyrid-4-yl)propanoic acid

A suspension of sodamide (14.8 g) in liquid ammonia (1.5 L) was prepared. To this was slowly added over 20 minutes 2-benzyloxy-4-methylpyridine (75.0 g) under nitrogen with a n-propanol-solid $CO_2$ condenser. The mixture was stirred for a further 40 minutes and sodium chloroacetate (44.1 g) was cautiously added over 8 minutes. The mixture was stirred for a further 90 minutes, quenched with ammonium chloride (20.3 g) and left to stir for 16 hours. The mixture was slowly quenched with water (1.2 L) and then washed with dichloromethane (600 ml). The aqueous layer was acidified to pH 4.5—5.0 with concentrated hydrochloric acid (approximately 50 ml) and on stirring at 0°C for 2 hours the title compound (60.4 g), m.p. 118—120°C (uncorrected), crystallised out.

Example 2

3-(2-Methoxypyrid-4-yl)propanoic acid

Sodamide (4.74 g) was added with stirring, under an isopropanol-solid $CO_2$ condenser, to liquid ammonia (250 ml). To this mixture was added 2-methoxy-4-methylpyridine (10.02 g). After 30 minutes the characteristic colour of the carbanion appeared and sodium bromoacetate (19.56 g) was added slowly. The reaction mixture was stirred for 2 hours, quenched with ammonium chloride (15.0 g) and left to stand to allow the ammonia to evaporate. Water (150 ml) was added to the residue and the solution was washed with dichloromethane (3 × 50 ml). The aqueous layer was taken to about pH 1 with concentrated hydrochloric acid, washed with ethyl acetate (3 × 50 ml), and taken to pH 4.5—5.0 with aqueous sodium hydroxide. A white solid precipitated, after one hour this was collected by filtration to give the title compound (10.2 g), m.p. 115—115.5°C (uncorrected). A further amount of title compound (0.91 g) was obtained from the mother liquors.

Example 3

3-(2-Methoxypyrid-4-yl)propanoic acid

Potassium (4.87 g) was added slowly with great care to liquid ammonia (250 ml) in a flask fitted with an isopropanol-solid $CO_2$ condenser. A few crystals of ferric nitrate were added to the flask after the addition of the first piece of potassium. The mixture was stirred for 15 minutes and 2-methoxy-4-methylpyridine (10.02 g) was added dropwise. The resultant mixture was stirred for 20 minutes and sodium bromoacetate (19.57 g) was added with stirring. Stirring was continued for 2 hours, ammonium chloride (15 g) was added and the mixture was left to stand for 16 hours to allow the ammonia to evaporate. Water (150 ml) was added and the title compound (10.45 g) was isolated as in Example 2. A further amount of product (1.03 g) was obtained from the mother liquors.

Example 4

3-(2-Methoxypyrid-4-yl)propanoic acid

Sodamide (1.9 kg) was charged to a vessel containing liquid ammonia (104 L). 2-Methoxy-4-methylpyridine (4.1 kg) was then added and the mixture was stirred for one hour. A sample was taken and found to be yellow, indicating that the carbanion had been formed. Sodium chloroacetate (7.56 kg) was added in 2 portions over 2 hours. The volume of ammonia in the vessel was periodically adjusted to about 100 L. Ammonium chloride (6.2 kg) was added in portions over 11 minutes. The mixture was left to evaporate overnight. The following day the remaining ammonia was evaporated off. Dichloromethane (40 L) was added followed by water (60 L). The organic layer was separated off. The extraction was repeated twice with dichloromethane (2 × 10 L). The aqueous layer was acidified to pH 1 with concentrated hydrochloric acid (16 L). The mixture was extracted with ethyl acetate (3 × 20 L) and then filtered to remove the ammonium chloride that had precipitated. The mixture was basified with 50% w/w aqueous sodium hydroxide solution to pH 4.5. The product precipitated, was filtered at −3°C, washed with water and dried at 50°C for 6 hours to give the title compound (3.47 kg).

Example 5

3-(2-Methoxypyrid-4-yl)propanoic acid

Sodamide (6.3 g) was added to liquid ammonia (330 ml) in a flask fitted with an isopropanol-solid $CO_2$ condenser. The mixture was stirred, 2-methoxy-4-methylpyridine (13.5 g) was added and the mixture was stirred for one hour, during which time the deep yellow colour associated with the carbanion developed. The phase transfer catalyst tris(dioxa 3,6 heptyl)amine, (36 g) was added, followed by sodium chloroacetate (12.45 g). After stirring for one hour further sodium chloroacetate (12.45 g) was added. After stirring for a further $1\frac{1}{2}$ hours the reaction mixture was quenched with ammonium chloride (20.6 g), and the mixture was stirred whilst the ammonia evaporated. Water (200 ml) was added to the residue and the title compound (8.9 g) was isolated as in Example 2.

Example 6

3-(2-Methoxypyrid-4-yl)propanoic acid

Sodamide (4.77 g) was added to liquid ammonia (200 ml) and tetrahydrofuran (50 ml) in a flask fitted with an isopropanol-solid $CO_2$ condenser. The resulting mixture was treated with 2-methoxy-4-methylpyridine (10.0 g). The reaction mixture was stirred for 40 minutes, during which time the characteristic colour of the carbanion developed. The reaction mixture was then treated with sodium chloroacetate (9.47 g). After stirring for two hours the reaction mixture was quenched with ammonium chloride (13.05 g) and the ammonia was allowed to evaporate overnight. Water (150 ml) was added, and the title compound (8.86 g) was isolated as described in Example 2.

In a similar reaction, using liquid ammonia (315 ml) and dioxan (105 ml) as solvent, 2-methoxy-4-methylpyridine (10.0 g) afforded the title compound (8.0 g).

Example 7

Methyl 3-(2-methoxypyrid-4-yl)propionate

3-(2-Methoxypyrid-4-yl)propanoic acid (4.44 kg) was added to methanol (36.0 L) containing concentrated sulphuric acid (2.23 L). The resulting solution was stirred and refluxed for $2\frac{1}{2}$ hours. After this time methanol (27.0 L) was distilled out and the residue was quenched in water (41.0 L). The pH of the aqueous mixture was adjusted to pH=10.0 using 0.880 ammonia (approximately 3.8 L). The aqueous layer was extracted with dichloromethane (4 × 12.0 L). The combined dichloromethane layers were distilled to remove the solvent, giving the title compound as an oil (4.08 kg). This compound is formylated with methyl formate and sodium hydride, according to the methods of EP—A—3677, to give methyl 2-formyl-3-(2-methoxypyrid-4-yl)propionate. Treatment of this with nitroguanidine and sodium methoxide, according to the methods of EP—A—3677, gives 2-nitroamino-5-(2-methoxypyrid-4-ylmethyl)pyrimidin-4-one.

**Claims for the Contracting States: BE CH DE FR GB IT LI LU NL SE**

1. A process for preparing a compound of the formula (II):

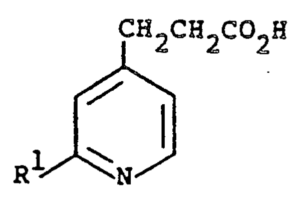

(II)

or a salt thereof, wherein $R^1$ is $C_{1-6}$alkoxy, benzyloxy or p-nitrobenzyloxy, which comprises reacting an anion of the formula (III):

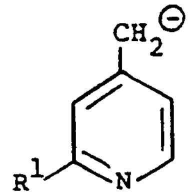

(III)

wherein $R^1$ is as hereinbefore defined with a compound of the formula (IV):

$$X—CH_2CO_2^-M^+ \quad (IV)$$

wherein X is a leaving group and $M^+$ is a counter-ion, and if desired interconverting an acid of the formula (II) with a salt thereof.

2. A process according to claim 1 wherein the anion of the formula (III) is derived from 2-methoxy-4-methylpyridine or 2-benzyloxy-4-methylpyridine.

3. A process according to claim 1 or 2 wherein X is chloro or bromo.

4. A process according to any one of claims 1 to 3 wherein the anion of the formula (III) has a sodium or potassium counter-ion.

5. A process according to any one of claims 1 to 4 wherein the compound of the formula (II) or salt thereof is subsequently esterified and formylated to give a compound of the formula (V):

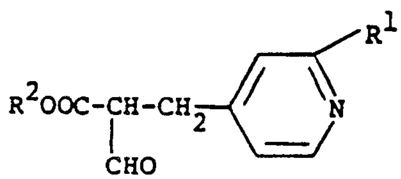

(V),

wherein $R^1$ is as defined in claim 1 and $R^2$ is $C_{1-6}$alkyl.

6. A compound of the formula (II):

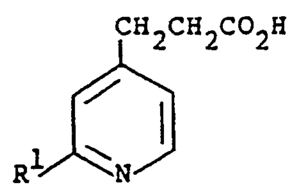

(II)

or a salt thereof, wherein $R^1$ is as defined in claim 1.

7. A compound according to claim 6 wherein $R^1$ is methoxy or benzyloxy.

8. A compound according to claim 6 which is 3-(2-Methoxypyrid-4-yl)propanoic acid.

**Claims for the Contracting State: AT**

1. A process for preparing a compound of the formula (II):

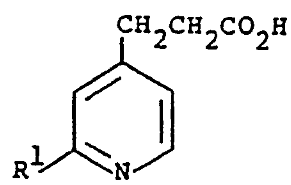

(II)

or a salt thereof, wherein $R^1$ is $C_{1-6}$alkoxy, benzyloxy or p-nitrobenzyloxy, which comprises reacting an anion of the formula (III):

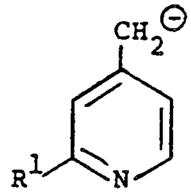

(III)

wherein $R^1$ is as hereinbefore defined with a compound of the formula (IV):

$$X—CH_2CO_2^-M^+ \qquad (IV)$$

wherein X is a leaving group and $M^+$ is a counter-ion, and if desired interconverting an acid of the formula (II) with a salt thereof.

2. A process according to claim 1 wherein the anion of the formula (III) is derived from 2-methoxy-4-methylpyridine or 2-benzyloxy-4-methylpyridine.

3. A process according to claim 1 or 2 wherein X is chloro or bromo.

4. A process according to any one of claims 1 to 3 wherein the anion of the formula (III) has a sodium or potassium counter-ion.

5. A process according to any one of claims 1 to 4 wherein the compound of the formula (II) or salt thereof is subsequently esterified and formylated to give a compound of the formula (V):

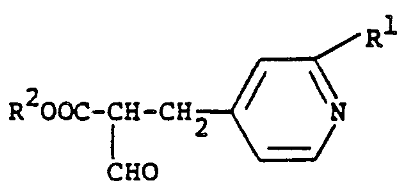

(V)

wherein $R^1$ is as defined in claim 1 and $R^2$ is $C_{1-6}$alkyl.

**Patentansprüche für die Vertragsstaaten: BE CH DE FR GB IT LI LU NL SE**

1. Verfahren zur Herstellung einer Verbindung der Formel (II)

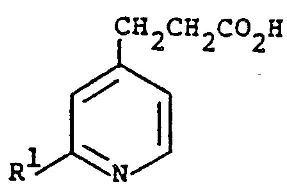

(II)

oder eines Salzes davon, in der $R^1$ einen $C_{1-6}$-Alkoxy-, Benzyloxy- oder p-Nitrobenzyloxyrest bedeutet, umfassend die Umsetzung eines Anions der Formel (III)

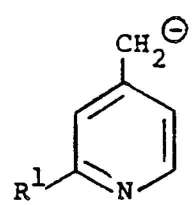

(III)

in der $R^1$ wie vorstehend definiert ist, mit einer Verbindung der Formel (IV)

$$X—CH_2CO_2^-M^+ \qquad (IV)$$

in der X ein austretender Rest ist und $M^+$ ein Gegenion bedeutet, und, gewünschtenfalls, Umwandlung der Säure der Formel (II) in ein Salz davon.

2. Verfahren nach Anspruch 1, wobei das Anion der Formel (III) von 2-Methoxy-4-methylpyridin oder 2-Benzyloxy-4-methylpyridin abgeleitet ist.

3. Verfahren nach Anspruch 1 oder 2, wobei X Chlor oder Brom ist.

4. Verfahren nach einem der Ansprüche 1 bis 3, wobei das Anion der Formel (III) ein Natrium- oder Kaliumgegenion aufweist.

5. Verfahren nach einem der Ansprüche 1 bis 4, wobei die Verbindung der Formel (II) oder das Salz davon anschließend zu einer Verbindung der Formel (V) verestert und formyliert wird:

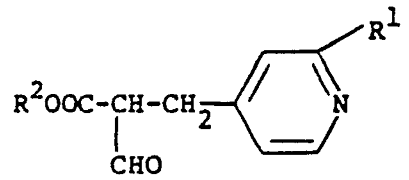

(V)

in der $R^1$ wie in Anspruch 1 definiert ist und $R^2$ einen $C_{1-6}$-Alkylrest bedeutet.

6. Eine Verbindung der Formel (II)

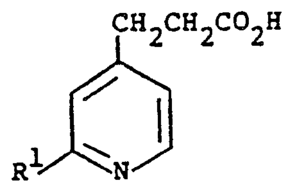

(II)

oder ein Salz davon, in der $R^1$ wie in Anspruch 1 definiert ist.

7. Verbindung nach Anspruch 6, in der $R^1$ eine Methoxy- oder Benzyloxygruppe ist.

8. Verbindung nach Anspruch 6, nämlich 3-(2-Methoxypyrid-4-yl)-propionsäure.

**Patentansprüche für den Vertragsstaat: AT**

1. Verfahren zur Herstellung einer Verbindung der Formel (II)

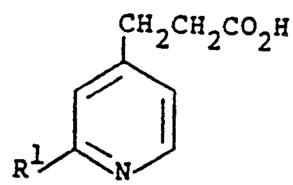

(II)

oder eines Salzes davon, in der $R^1$ einen $C_{1-6}$-Alkoxy-, Benzyloxy- oder p-Nitrobenzyloxyrest bedeutet, umfassend die Umsetzung eines Anions der Formel (III)

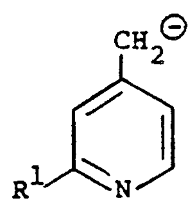

(III)

in der $R^1$ wie vorstehend definiert ist, mit einer Verbindung der Formel (IV)

$$X{-}CH_2CO_2^-M^+ \qquad (IV)$$

in der X ein austretender Rest ist und $M^+$ ein Gegenion bedeutet, und, gewünschtenfalls, Umwandlung der Säure der Formel (II) in ein Salz davon.

2. Verfahren nach Anspruch 1, wobei das Anion der Formel (III) von 2-Methoxy-4-methylpyridin oder 2-Benzyloxy-4-methylpyridin abgeleitet ist.

3. Verfahren nach Anspruch 1 oder 2, wobei X Chlor oder Brom ist.

4. Verfahren nach einem der Ansprüche 1 bis 3, wobei das Anion der Formel (III) ein Natrium- oder Kaliumgegenion aufweist.

5. Verfahren nach einem der Ansprüche 1 bis 4, wobei die Verbindung der Formel (II) oder das Salz

davon anschließend zu einer Verbindung der Formel (V) verestert und formyliert wird:

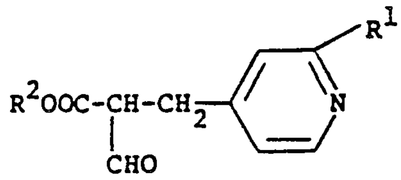

(V)

in der $R^1$ wie in Anspruch 1 definiert ist und $R^2$ einen $C_{1-6}$-Alkylrest bedeutet.

**Revendications pour les Etats contractants: BE CH DE FR GB IT LI LU NL SE**

1. Procédé pour préparer un composé de la formule (II):

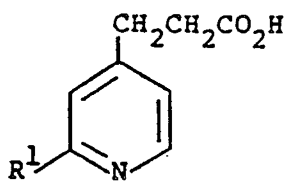

(II)

ou un de ses sels, dans laquelle $R^1$ est un alkoxy en $C_{1-6}$, benzyloxy ou p-nitrobenzyloxy, qui comprend la réaction d'un anion de la formule (III):

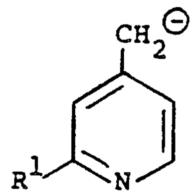

(III)

où $R^1$ est tel que défini plus haut, avec un composé de la formule (IV):

$$X—CH_2CO_2^-M^+ \qquad (IV)$$

où X est un groupement qui est éliminé et $M^+$ est un contre-ion et, si on le désire, la conversion d'un acide de la formule (II) en un de ses sels.

2. Procédé suivant la revendication 1 dans lequel l'anion de la formule (III) est dérivé de la 2-méthoxy-4-méthylpyridine ou de la 2-benzyloxy-4-méthylpyridine.

3. Procédé suivant la revendication 1 ou 2 dans lequel X est chloro ou bromo.

4. Procédé suivant l'une quelconque des revendications 1 à 3 dans lequel l'anion de la formule (III) a un contre-ion sodium ou potassium.

5. Procédé suivant l'une quelconque des revendications 1 à 4 dans lequel le composé de la formule (II) ou un de ses sels est ensuite estérifié et formylé pour donner un composé de la formule (V):

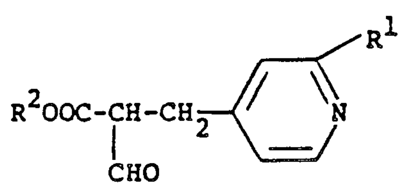

(V)

où $R^1$ est tel que défini dans la revendication 1 et $R^2$ est un alkyle en $C_{1-6}$.

6. Composé de la formule (II):

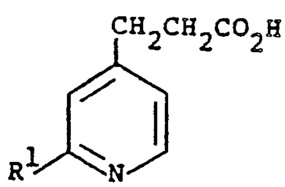

(II)

ou un de ses sels où $R^1$ est tel que défini dans la revendication 1.

7. Composé suivant la revendication 6 dans lequel $R^1$ est méthoxy ou benzyloxy.

8. Composé suivant la revendication 6 qui est l'acide 3-(2-méthoxypyrid-4-yl)propanoïque.

**Revendications pour l'Etat contractant: AT**

1. Procédé pour préparer un composé de la formule (II):

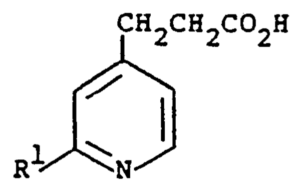

(II)

ou un de ses sels, dans laquelle $R^1$ est un alkoxy en $C_{1-6}$, benzyloxy ou p-nitrobenzyloxy, qui comprend la réaction d'un anion de la formule (III):

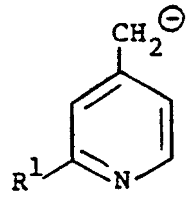

(III)

où $R^1$ est tel que défini plus haut, avec un composé de la formule (IV):

$$X{-}CH_2CO_2^-M^+ \qquad (IV)$$

où X est un groupement qui est éliminé et $M^+$ est un contre-ion et, si on le désire, la conversion d'un acide de la formule (II) en un de ses sels.

2. Procédé suivant la revendication 1 dans lequel l'anion de la formule (III) est dérivé de la 2-méthoxy-4-méthylpyridine ou de la 2-benzyloxy-4-méthylpyridine.

3. Procédé suivant la revendication 1 ou 2 dans lequel X est chloro ou bromo.

4. Procédé suivant l'une quelconque des revendications 1 à 3 dans lequel l'anion de la formule (III) a un contre-ion sodium ou potassium.

5. Procédé suivant l'une quelconque des revendications 1 à 4 dans lequel le composé de la formule (II) ou un de ses sels est ensuite estérifié et formylé pour donner un composé de la formule (V):

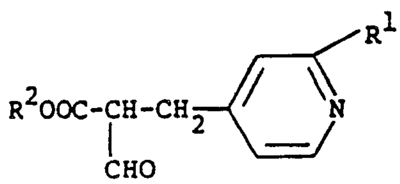

(V)

où $R^1$ est tel que défini dans la revendication 1 et $R^2$ est un alkyle en $C_{1-6}$.